# EUROPÄISCHE PATENTSCHRIFT

(11) **EP 1 861 152 B1**
(45) Veröffentlichungstag und Bekanntmachung des Hinweises auf die Patenterteilung: **12.09.2018**
(21) Anmeldenummer: 06724889.8
(22) Anmeldetag: 24.02.2006
(51) Int. Cl.: A61M 16/04, A61L 29/00, A61L 31/00

(54) **VORRICHTUNG ZUR ATEMLUFTZUFÜHRUNG**
INHALED AIR SUPPLYING DEVICE
DISPOSITIF D'AMENEE D'AIR RESPIRATOIRE

(30) Priorität: 28.02.2005 DE 102005009577; 31.03.2005 DE 102005015045
(43) Veröffentlichungstag der Anmeldung: 05.12.2007
(73) Patentinhaber: Tracoe medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2006/060282
(87) Internationale Veröffentlichungsnummer: WO 2006/089961

(56) Entgegenhaltungen:
- WO-A-2004/096330
- DE-A1- 3 921 524
- DE-A1- 19 855 521
- US-A1- 2003 066 532
- US-A1- 2003 213 492
- US-A1- 2004 112 388
- US-A1- 2004 236 365
- US-A1- 2005 015 105

## Beschreibung

Die vorliegende Erfindung betrifft eine Vorrichtung zur Atemluftzuführung mit mindestens einem Tubus.

Aus dem Stand der Technik sind verschiedene Arten von Vorrichtungen zur Atemluftzuführung bekannt. Solche Vorrichtungen finden vor allem als sogenannte Endotrachealtuben zur künstlichen Beatmung von Patienten in der Intensiv- und Operationsmedizin Verwendung. Dabei wird der Endotrachealtubus durch die Nase bzw. den Mund des Patienten bis in die Luftröhre bzw. den oberen Bronchialbereich geführt.

Darüber hinaus werden derartige Vorrichtungen als sogenannte Tracheostomiekanülen zur Beatmung von Patienten mit Luftröhrenschnitt verwendet. Diese Tracheostomiekanülen können auch zur dauerhaften selbständigen Atmung des Patienten eingesetzt werden, z.B. nach Kehlkopfoperationen. Dabei werden die Tracheostomiekanülen durch den Luftröhrenschnitt in die Luftröhre eingeführt, wo sie sich ebenfalls bis in den oberen Bronchialbereich erstrecken.

Die Tuben bilden jeweils ein durchgehendes Lumen, welches den Brochialbereich des Patienten mit der Umgebungsluft oder auch mit einem Beatmungsautomaten verbinden.

Die aus dem Stand der Technik bekannten Vorrichtungen bestehen zumeist aus verschiedenen Kunststoffen, die flexibel sind sowie preiswert unter sterilen Bedingungen herstellbar sind. Die aus dem Stand der Technik bekannten Tuben weisen jedoch den Nachteil auf, daß sie aufgrund der Beschaffenheit der Kunststoffmaterialien einen hohen Reibungskoeffizienten aufweisen. Dies ist insbesondere bei der Intubation, d.h. der Einführung des Tubus durch den Mund, die Nase oder durch den Luftröhrenschnitt nachteilig. Aufgrund der erhöhten Reibung zwischen den Vorrichtungen und den beteiligten Organen des Patienten ist bei der Intubation ein erheblicher Kraftaufwand von Ärzten und Pflegern erforderlich. Darüber hinaus kann es in Folge der Reibung zu Verletzungen der beteiligten Organe kommen.

Die für die Herstellung der Tuben verwendeten Kunststoffmaterialien nehmen Verunreinigungen, insbesondere mit Speichel vermengte Sekretionen und Speisereste schnell an und verschmutzen an ihrer Oberfläche. Dabei kommt es insbesondere bei längerer Beatmung über die Tuben zu einem Verkleben bzw. Verkrusten der Tuben. Die sich an der Oberfläche der Tuben anlagernden Verunreinigungen bilden den Nährboden für bakterielle Infektionen der Atemwegsorgane.

Eine häufige Komplikation bei der (Be-) Atmung von Patienten über Tuben der zuvor beschriebenen Art ist die sogenannte Aspiration. Dabei dringen infolge der durch die in die Atemwege eingeführten Tuben ausgeschaltete Schutzreflexe flüssige oder feste Stoffe in die Atemwege ein, wodurch es zu Verlegungen der Atemwege und in Folge dessen zu Hypoxien kommt. Darüber hinaus bilden die Verunreinigungen im Bereich der Bronchien Brutstätten für Bakterien und Keime, so daß bei der Beatmung über Tuben sehr häufig Pneumonien zu den Folgeerkrankungen der Beatmung gehören.

Um während der Atemluftversorgung über einen Tubus eine Aspiration, d.h. das Eindringen von flüssigen und festen Stoffen in den Bronchialbereich, zu verhindern, sind aus dem Stand der Technik, beispielsweise der EP 0 930 909 B1 sogenannte "Cuffs" bekannt. Diese sind aufblasbare oder mit einem anderen Fluid befüllbare flexible Manschetten, welche an der Außenseite des Tubus vorgesehen und im allgemeinen fest mit diesem verbunden sind. Die Manschetten sind so am Tubus angeordnet, daß sie im oberen Bereich der Luftröhre zu liegen kommen. Im aufgeblasenen bzw. befüllten Zustand füllen sie die Luftröhre außerhalb des Tubus aus und dichten die Außenseite des Tubus gegenüber der Wand der Luftröhre ab und verhindern so, daß Flüssigkeiten oder feste Stoffe, z.B. Speisereste, durch das Lumen zwischen der Außenwand des Tubus und der Wand der Speiseröhre hindurch bis in den Bereich der Bronchien gelangen.

Die aus dem Stand der Technik bekannten Niederdruckmanschetten haben gewisse Nachteile, da sie, um eine Abdichtung zu ermöglichen, einen Durchmesser aufweisen müssen, welcher grundsätzlich größer sein muß als der Innendurchmesser der Luftröhre. Dabei kommt es bei befüllter Manschette zur Bildung von Falten im Material der Manschette, welche sich vom Außenumfang der Manschette radial nach innen erstrecken. Diese Falten bilden Durchlaßkanäle, durch welche Flüssigkeiten bis in den Bronchialbereich gelangen können.

Dabei hat sich herausgestellt, daß sich die Falten bevorzugt in Längsrichtung der Manschette erstrecken, wobei sich auch Falten überkreuzen oder aneinander anschließen können, was im Ergebnis jedenfalls dazu führt, daß sich zwischen diesen Faltenwänden kleine Zwischenräume bilden, die sich aufgrund der begrenzten Duktilität des Folienmaterials, auch wenn dieses hochflexibel ist, nicht vollständig schließen. Das heißt, insbesondere am radial inneren Ende der Falten bilden sich sogenannte "Faltenösen", die eine gewisse Durchlässigkeit für Flüssigkeiten und somit auch für die sich in der Trachea ansammelnden Sekrete haben, die somit in die Lunge gelangen können und dabei sehr häufig Pneumonien auslösen. Auch am Übergang derartiger Falten zu der Wand der Trachea bilden sich in etwa dreieckige Zwickel, die Durchgangsöffnungen bzw. -kanäle zwischen Trachea und Manschette und entlang der Außenseite der Manschette definieren.

Aus diesem Grund besteht eine Tendenz in Richtung für Manschetten mit noch höherer Flexibilität, um derartige Durchgangskanäle zu vermeiden bzw. noch enger und damit undurchlässiger zu machen, was man im allgemeinen durch eine Verringerung der Wandstärke der Manschette erreicht, wobei bereits Folien mit Wandstärken von nur 5 µm verwendet werden.

Es kommt allerdings vor, daß sich die Manschetten mit Wasser/Sekret füllen. Je dünner die Manschetten sind, desto rascher kann die Flüssigkeit durch die Manschettenwand hindurchtreten (diffundieren/migrieren). Dies gilt auch, wenn die Manschetten möglicherweise sehr enge Falten bilden, durch die kaum noch Sekret hindurchtritt.

Hinzu kommt das Problem, daß das extrem dünnwandige Manschettenmaterial schwierig zu handhaben ist und auch leicht beschädigt werden kann.

Aus der WO 2004/096330 A2 ist eine Vorrichtung zum Zuführen eines Fluids, wie beispielsweise eine Endotrachealkanüle, bekannt. Die Endotrachealkanüle weist eine Manschette mit einer hydrophilen Beschichtung auf.

Die DE 198 55 521 A1 zeigt einen Tubus zum Einführen in die Luftröhre eines Patienten, mit einem zentralen Lumen zur Beatmung bzw. zur Atmung und mit mindestens einer aufblasbaren, an der Außenseite des Tubus vorgesehenen Manschette, welche der Abdichtung der Tubusaußenseite gegenüber der Wand der Luftröhre dient. Die Manschette weist auf ihrer äußeren Oberfläche eine Beschichtung auf, welche quellfähig und/oder begrenzt fließfähig ist.

Die DE 39 21 524 A1 beschreibt einen Trachealtubus, der im Bereich seiner Spitze eine Blockungsmanschette aufweist. Mindestens eine Materialschicht der Blockungsmanschette ist narkosegasundurch lässig.

Die US 2004/0236365 A1 beschreibt einen Katheter zum Einbringen in Körperöffnungen. Der offenbarte Blasenkatheter weist einen oder mehrere entfaltbare Blockungsmanschetten mit einer hydrophilen Beschichtung auf.

Die US 2004/0112388 A1 beschreibt eine Laryngeal-Maske mit einer hydrophilen Beschichtung.

Gegenüber diesem Stand der Technik liegt der vorliegenden Erfindung die Aufgabe zugrunde, Vorrichtungen zur Atemluftzuführung bereitzustellen, welche die vorgenannten Nachteile vermeiden.

Erfindungsgemäß wird diese Aufgabe dadurch gelöst, daß eine Vorrichtung zur Atemluftzuführung mit mindestens einem Tubus und mindestens einer mit einem Fluid befüllbaren und an der Außenseite des Tubus vorgesehenen Manschette zur Abdichtung des Tubus gegenüber der Wand der Luftröhre bereitgestellt wird, wobei mindestens die Manschette mindestens abschnittsweise eine die Oberfläche des Materials der Vorrichtung verändernde, abriebfeste Oberflächenschicht (6, 7) mit einer Schichtdicke von weniger als 10 µm aufweist

Auf diese Weise bleiben die Vorteile des verwendeten Grundmaterials, wie z. B. Flexibilität und preiswerte Herstellung, weiterhin erhalten, während die Oberflächeneigenschaften der Kunststoffelemente der Vorrichtung mit Hilfe geeigneter Ausbildung der Oberflächenschicht, insbesondere durch Beschichtung, gezielt verändert und anwendungsbezogen eingestellt werden.

Zweckmäßigerweise ist die Manschette eine sogenannte "high volume low pressure"-Manschette. Diese zeichnet sich dadurch aus, daß sie nur eine geringe Wandstärke aufweist und der Außendurchmesser des Manschettenballons größer ist als der Durchmesser der Luftröhre. Beim Füllen paßt sich die Manschette daher unter Ausbildung von Falten den Konturen der Luftröhre an. Eine Abdichtung durch diese Manschetten ist bereits bei verhältnismäßig geringen Drücken (typischerweise 20 Millibar) möglich. Aufgrund der geringen Drücke wird eine Schädigung der Luftröhre vermieden.

Insbesondere ist es vorteilhaft, wenn die Manschette aus einer flexiblen, dünnwandigen Kunststoffolie besteht. Dabei sind Ausführungsformen bevorzugt, bei welchen die Kunststoffolie den Tubus in Form eines aufgeweiteten Schlauchabschnitts umgreift. Insbesondere bevorzugt ist eine Ausführungsform der Erfindung, bei welcher die Folie auf mindestens einer ihrer Seiten eine wasserundurchlässige Schicht aufweist.

Die Beschichtung mit einem wasserundurchlässigen Material ermöglicht es, für die Manschette dennoch sehr dünnwandiges Folienmaterial zu verwenden, das hochflexibel ist und extrem kleine Faltenzwischenräume bildet, und das seinerseits dennoch nicht selbst wasser- bzw. wasserdampfdurchlässig ist. Der Begriff "wasserundurchlässig", der auch "wasserdampfundurchlässig" einschließen soll, ist dabei selbstverständlich in dem gegebnen technischen Zusammenhang zu interpretieren, d. h. eine gewisse verbleibende, minimale Wasserdurchlässigkeit, die für den praktischen Gebrauch toleriert werden kann, mag nach wie vor vorhanden sein. Wesentlich ist aber, das die Wasserdurchlässigkeit z. B. einer weichen Polyurethanfolie von 5 µ Wandstärke auf einen Bruchteil von weniger als 1/3, vorzugsweise auf weniger als 1/10 oder gar unter 1/100 reduziert wird. Bei einer wasserundurchlässigen Schicht kommt es hingegen nicht auf die Dicke der Schicht an, so daß diese auch dicker als 10 µm sein kann, wobei die Abriebfestigkeit der Schicht lediglich eine bevorzugte Ausführungsform darstellt.

Das Folienmaterial kann dabei ausgewählt werden aus einer Gruppe, die besteht aus Polyurethan, Polyester, PET und PVC, wobei Polyurethan am meisten bevorzugt ist, weil es zum einen ein sehr gewebeverträgliches Material ist und zum anderen auch in sehr weicher und duktiler Form hergestellt werden kann. Je weicher und duktiler das Folienmaterial ist, desto kleiner sind bei gegebenener Wandstärke auch die entstehenden Faltenösen oder Zwickel am Übergang zwischen der Falte einer Manschette und der Trachea. Polyurethan ist beispielsweise in unterschiedlichen Härten herstellbar. Dabei haben allerdings besonders weiche Materialien den Nachteil, daß sie auch an Reißfestigkeit verlieren, so daß insofern ein Kompromiß zwischen Reißfestigkeit und Duktilität geschlossen werden muß. Der Nachteil der relativ hohen Wasser- bzw. Wasserdampfdurchlässigkeit von dünnwandigem (und womöglich auch weichem) Polyurethan wird dabei durch die erfindungsgemäßen Maßnahmen problemlos ausgeglichen.

Dabei ist es zweckmäßig, wenn die Manschette auf ihrer dem Tubus abgewandten Außenseite mit dem wasserundurchlässigen Material beschichtet ist.

Zweckmäßigerweise sollte die Folie eine Wandstärke von weniger als 100 µm haben, wobei andererseits auch Wandstärken von mehr als 5 µm aus praktischen Gründen bevorzugt werden. Wandstärken von 10 µm bis 50 µm haben sich in der Praxis als geeignet erwiesen, wobei eine Wandstärke der Folie im Bereich von 15 bis 30 µm für die vorliegende Erfindung am meisten bevorzugt ist.

In einer besonders bevorzugten Ausführungsform der vorliegenden Erfindung ist darüber hinaus vorgesehen, daß eine zusätzliche, hydrophobisierende Schicht auf der Außenseite der Manschette bzw. des Folienmaterials, aus welchem die Manschette besteht, aufgebracht ist. Dabei wird eine solche zusätzliche, hydrophobisierende Schicht z. B. auf einer bereits aufgebrachten wasserundurchlässigen Beschichtung aufgebracht oder aber die wasserundurchlässige Beschichtung befindet sich auf der anderen Seite der Folie, die als Innenseite der Manschette dient.

Im Idealfall lassen sich auch Beschichtungsmaterialien finden, die eine gute Gewebeverträglichkeit haben, gut auf dem Folienmaterial, insbesondere Polyurethan, haften und schließlich auch sowohl wasserundurchlässig als auch hydrophob sind.

Materialien, die als wasserundurchlässige Beschichtungen in Frage kommen, stehen in einer sehr vielfältigen Form zur Verfügung. Insbesondere sind zahlreichen anorganische Beschichtungen hierfür geeignet, wie z. B. Metalle oder Siliciumdioxid, und andererseits auch organische Beschichtungen, wie z. B. fluorierte Kohlenwasserstoffe wie Teflon oder Silan. Wenn die Beschichtung auf der Innenseite der Manschette erfolgt oder aber durch eine zusätzlich aufgebrachte hydrophobe Schicht abgedeckt wird, spielt auch die Gewebeverträglichkeit oder Körperverträglichkeit nur eine geringe Rolle, obwohl es grundsätzlich bevorzugt ist, dennoch körperverträgliche und ungiftige Beschichtungsmaterialien zu verwenden, selbst wenn diese in der Regel nicht mit Körperteilen in Berührung kommen.

Insbesondere die Kombination einer wasserundurchlässigen mit einer hydrophoben Beschichtung hat den Vorteil, daß man nicht notwendigerweise auf die Verwendung extrem dünnwandiger Folien im Bereich von 5 µm oder gar noch darunter angewiesen ist, da durch die hydrophobe Beschichtung ein Hindurchtreten von flüssigem Sekret durch die von den Falten gebildeten Zwischenräume selbst dann verhindert wird, wenn diese etwas größere Abmessungen haben, wie es bei der Verwendung entsprechend dickerer Folien unvermeidlich erscheint.

Dabei hat sich herausgestellt, daß Folienwandstärken in der Größenordnung von ca. 25 µm (genauer gesagt im Bereich von 15 bis 30 µm) sehr gut handhabbar sind und bei einer entsprechenden, kombinierten Beschichtung mit einem wasserundurchlässigen und einem hydrophoben Material zu Manschetten führen, die im Vergleich zu allen bekannten Manschetten die günstigsten Eigenschaften aufweisen, was zum einen ihre Handhabbarkeit und Sicherheit (zum Beispiel gegen unbeabsichtigte Beschädigungen), zum anderen aber auch ihre Funktion betrifft. Diese Manschetten sind dünnwandig und flexibel genug, um sich bei einem geringen Überdruck dicht an die Wand der Trachea anzulegen, wobei die entstehenden Falten hinreichend klein sind, um zumindest aufgrund der zusätzlichen hydrophoben Beschichtung den Durchtritt von Sekret durch die Faltenzwischenräume zu verhindern.

Dabei können sowohl die wasserundurchlässigen Beschichtungen als auch die hydrophoben Beschichtungen in sehr geringen Schichtdicken aufgebracht werden, die die mechanischen Eigenschaften der Folien und insbesondere deren Flexibilität und Duktilität nicht negativ beeinflussen. Vorzugsweise beträgt die Dicke der wasserundurchlässigen Schicht weniger als 5 µm, insbesondere weniger als 1 µm und besonders bevorzugt weniger als 200 nm. Das gleiche gilt auch für die hydrophobe Beschichtung, so daß, wenn jede der beiden Schichten beispielsweise nur etwa 100 nm dick ist, die gesamte Dicke der Beschichtungen bei einer Folie mit einer Wandstärke von 20 µm nur 1 % ausmacht und damit für die mechanischen Eigenschaften der Folie vernachlässigbar ist.

Vor allem wird beispielsweise durch eine hydrophobe Oberflächenschicht der vorstehend beschriebenen Manschetten verhindert, daß trotz einer gewissen Faltenbildung das Sekret, welches sich oberhalb der Manschette ansammelt, in die unvermeidbaren Falten der Manschetten bzw. durch diese hindurch dringt, und zwar selbst dann, wenn aufgrund der maximalen lichten Weite dieser Falten das Sekret leicht entlang dieser Falten an der Manschette vorbei fließen würde, wenn deren Oberfläche nicht aus einem besonderen Material insbesondere einem hydrophoben Maetrial bestehen würde.

Eine besondere Oberflächenschicht ist auch zweckmäßig bei einer Manschette, die aus einem elastischem Material besteht, das sich beim Befüllen ausdehnt und sich dabei ohne Faltenbildung der Kontur der Luftröhre optimal anpaßt. Da auch eine solche Manschette keinen starken Druck auf die Wand der Trachea ausüben darf und auch insofern die latente Gefahr einer Undichtigkeit besteht., kann die Dichtigkeit einer solchen Manschette in der Trachea durch die angepaßte Oberflächenschicht weiter verbessert werden.

Bevorzugt ist eine Ausführungsform der Erfindung, bei der die Oberflächenschichten dünn sind, insbesondere eine Dicke von weniger als 5 µm, vorzugsweise weniger als 3 µm und besonders bevorzugt weniger als 1 µm, aufweisen. Für entscheidende Änderungen der Oberflächen reichen sehr düne Schichtdicken von sogar weniger als 500 oder 200 nm ohne weiteres aus. Oft genügen hierfür Schichtstärken unter 100 nm oder sogar monomolekulare bzw. monoatomare Lagen von wenigen nm Stärke, welche die untere Grenze der Schichtdicke bei etwa 2 bis 5 nm definieren. Dünne Oberflächenschichten haben den Vorteil, daß sie die Flexibilität und/oder Elastizität des Grundmaterials im wesentlichen unbeeinflusst lassen. Dies ist insbesondere bei den zuvor genannten "high volume low pressure"-Manschetten vorteilhaft, bei welchen bereits das Material der Manschette zumeist weniger als 50 µm dick ist.

Die beanspruchte Abriebfestigkeit soll vor allem bei einem normalen Gebrauch beim Berühren, Anfassen, Sterilisieren und Einsetzen gegeben sein. Sie ist insbesondere bei einer chemischen oder physikalischen Bindung der Oberflächenschicht an dem Kunststoffmaterial der Vorrichtung gegeben. Diese Eigenschaft soll die erfindungsgemäßen Oberflächenschichten vor allem von Cremes oder Gelen sowie quellfähigen Beschichtungen unterscheiden, welche auf die Oberfläche der Tuben mit relativ geringer Haftung aufgebracht sind, so daß bei deren Handhabung Maßnahmen getroffen werden müssen, um die Integrität der Beschichtung nicht zu beeinträchtigen. Cremes oder Gele beispielsweise lassen sich nach dem Auftragen wieder abwischen, bzw. abreiben und zumindest lokal weitgehend entfernen. Diese Nachteile sollen durch die Abriebfestigkeit vermieden werden

Die vorgenannten abriebfesten Oberflächenschichten lassen sich heute in Dünnschichttechnologie auf verschiedene Weisen preiswert und rationell herstellen.

Besonders bevorzugt ist eine Ausführungsform der Erfindung, bei der die Oberflächenschicht eine Plasmabeschichtung ist.

Ist die Oberflächenschicht bzw. Beschichtung ein Polymer, so kann das Polymer bereits während des Herstellungsprozesses dem Grundmaterial zugeführt werden. Verwendet man hierzu Polymere bzw. Copolymere des Kunststoffmaterials der Vorrichtung, welche eine geringere Oberflächenspannung als das Kunststoffmaterial aufweisen, so kommt es bereits in der Schmelze zu einer Anreicherung des zugesetzten Polymers bzw. Copolymers an der Oberfläche. Auf diese Weise werden dünne Oberflächeschichten mit den Oberflächeneigenschaften des Polymers bzw. Copolymers gebildet.

Darüber hinaus ist es vorteilhaft, wenn die Oberflächenschicht gegenüber dem Material der Vorrichtung einen verringerten Reibungskoeffizienten aufweist. Dies ermöglicht eine Einführung der Vorrichtung in die Atemwegsorgane des Patienten mit verringerter Kraft, wodurch insbesondere auch das Verletzungsrisiko für den Patienten minimiert wird.

In einer weiteren vorteilhaften Ausgestaltung weist die erfindungsgemäße Vorrichtung eine Silber enthaltende Beschichtung auf, die im einfachsten Fall beispielsweise durch Bedampfung mit elementarem Silber hergestellt wird. Alternative könnte Silber bzw. könnten Silberionen in ein Beschichtungsmaterial, insbesondere in hydrophobes Beschichtungsmaterial eingebaut sein. Bekanntermaßen hat Silber eine antibakterielle Wirkung, die man sich auf diese Weise zunutze macht. Dabei kann eine aufgedampfte Silberschicht so dünn sein, daß sie die hydrophoben Eigenschaften einer darunter liegenden, hydrophoben Schicht nicht nachteilig beeinflusst. Die Hydrophobie der Oberflächenschicht bleibt also auch im Falle einer Silberbedampfung erhalten.

In einer bevorzugten Ausführungsform der Erfindung ist die Oberflächenschicht schmutzabweisend, so daß sich keine Schmutzablagerungen an der Oberfläche der Vorrichtung, d.h. insbesondere des Tubus und/oder der Manschette, anlagern können, welche als Brutstätte für Bakterien dienen können.

Besonders bevorzugt ist eine Ausführungsform der Erfindung, bei der die Oberflächenschicht selbstreinigend ist. Solche selbstreinigenden Oberflächenschichten sind beispielsweise aus der Bautechnik bekannt, wo sie es ermöglichen, den Reinigungsbedarf von Glasflächen erheblich zu reduzieren. Für eine solche selbstreinigende Oberflächenschicht kann es neben der Auswahl des geeigneten Oberflächenschichtmaterials erforderlich sein, die Oberfläche mit einer Mikrostrukturierung zu versehen. Dies kann beispielsweise durch ein Verfahren geschehen, bei dem sich eine Selbstordnung der zur Beschichtung bzw. als Oberflächenschicht vorgesehenen Moleküle an der Oberfläche des Tubus oder der Manschette einstellt.

Bevorzugt ist eine Ausführungsform der Erfindung, bei der das Oberflächenschichtmaterial ein fluorhaltiges, z.B. CF₄, C₂F₆ u.a., oder ein silikonhaltiges Material ist. Beispielhaft seien hier Beschichtungen bzw. Oberflächenschichten, genannt die unter Verwendung von Silanen, z.B. Tetramethylsilan, Siloxanen oder deren Polymeren hergestellt werden.

Die Beschichtungen können auch durch chemische Bindungen (grafting) an der Oberfläche des Kunststoffmaterials der Vorrichtung verankert sein.

Die erfindungsgemäßen Oberflächenschichten modifizieren nicht nur die Oberflächeneigenschaften der Vorrichtung in der gewünschten Weise, sondern sie bilden z. B. auch eine Diffusionsbarriere für eventuell im Grundmaterial des Tubus oder der Manschette enthaltene Zusatzstoffe wie Weichmacher und dergleichen. Auf diese Weise gelangen geringere Mengen dieser Substanzen in den Organismus des Patienten.

Die zuvor genannten Oberflächeneigenschaften der Oberflächenschichtsmaterialien können wahlweise einzeln oder in Kombination zweckmäßig sein.

Bevorzugt ist eine Ausführungsform der Erfindung, bei der die Manschette und der Tubus beschichtet sind, wobei diese Beschichtungen sich voneinander unterscheiden können. Jedoch kann es alternativ zweckmäßig sein, wenn nur eines der Elemente oder ausgewählte Abschnitte davon beschichtet sind. Auch eine Beschichtung der Innenwände des Tubus oder der Manschette ist möglich, um beispielsweise ein Verkrusten des Tubus und/oder ein Verkleben der Wände der Manschette zu verhindern.

Besonders bevorzugt ist eine Ausführungsform der Erfindung, bei der die Vorrichtung wahlweise eine Tracheostomiekanüle, ein Endotrachealtubus, eine Larynxmaske oder ein Pharyngealtubus ist.

Weitere Merkmale, Vorteile und Anwendungen der vorliegenden Erfindung werden anhand der folgenden Beschreibung einer bevorzugten Ausführungsform sowie der dazugehörigen Figuren deutlich.
- Figur 1: zeigt schematisch eine erfindungsgemäße Vorrichtung mit einer aufgeblasenen Manschette.
- Figur 2: zeigt eine Schnittansicht mit Blickrichtung entlang der Achse des Tubus und mit einer Schnittebene, die etwa entlang der in Figur 1 mit II-II bezeichneten, strichpunktierten Linie verläuft.
- Figur 3: zeigt einen vergrößerten Ausschnitt entsprechend dem mit III bezeichneten Kreis in Figur 2.
- Figur 4: zeigt einen nochmals vergrößerten Schnitt durch die Wand des Folienmaterials der Manschette.
- Figur 5: zeigt einen vergrößerten Ausschnitt entsprechend dem mit III bezeichneten Kreis in Figur 2, wobei die Berührung mit der Trachea dargestellt ist.

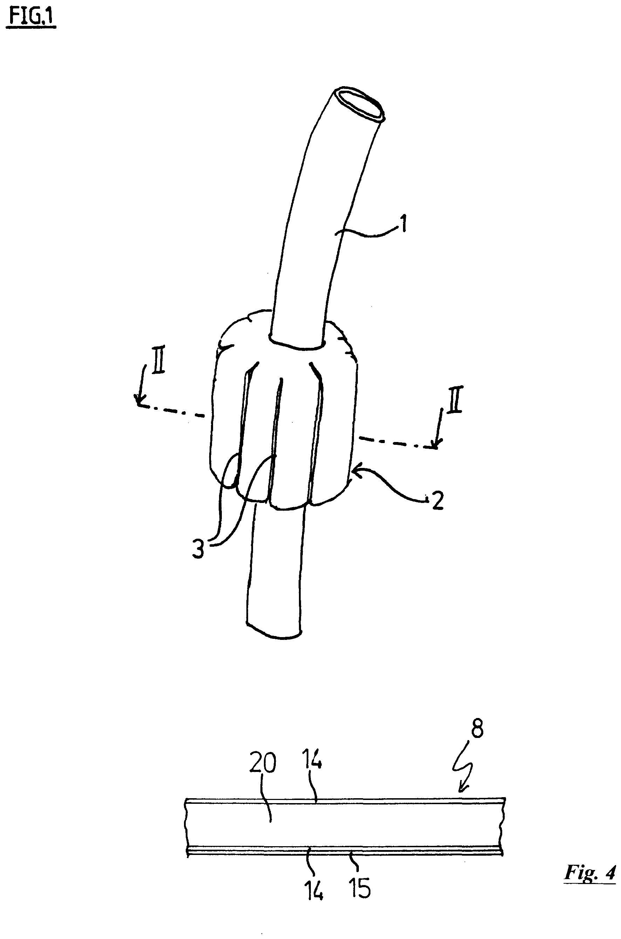

Man erkennt in Figur 1 eine Tracheostomiekanüle, die im wesentlichen aus einem Schlauch bzw. einem Rohr 1 besteht, an dessen äußerem Umfang in einem mittleren Abschnitt eine aufblasbare Manschette 2 vorgesehen ist. Die Manschette 2 ist mit dem Tubus fest verschweißt. Sie kann alternativ aber auch angeklebt oder einstückig mit dem Tubus 1 hergestellt sein. Ein in der Wand des Tubus 1 vorgesehener, hier nicht erkennbarer Kanal hat in der Außenwand des Tubus 1 eine Öffnung im Bereich der Manschette 2, so daß die Manschette 2 über diese Austrittsöffnung des in der Wand des Tubus 1 vorgesehenen Kanals aufblasbar, gegebenenfalls aber auch entlüftbar ist. Die Atmung erfolgt über das zentrale Lumen 4 des Tubus 1.

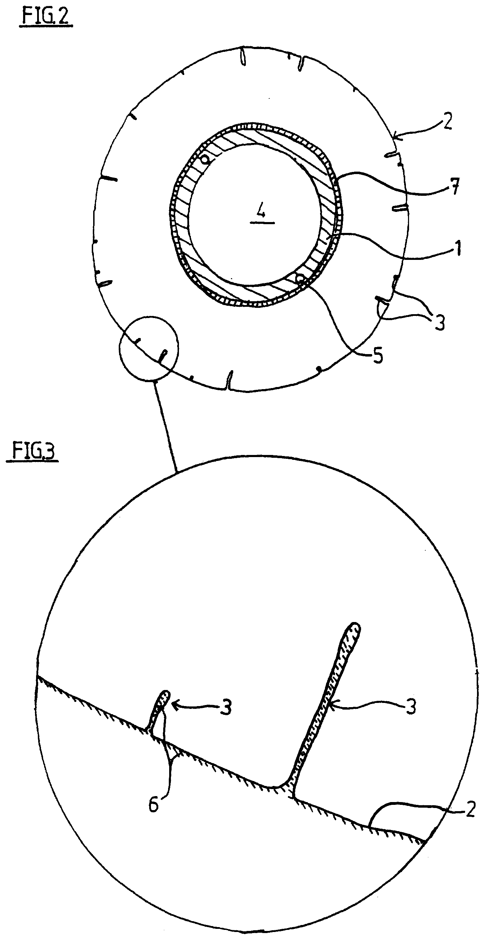

In Figur 2 ist der Tubus im Bereich der Manschette 2 im Schnitt dargestellt. Man erkennt schraffiert die Wand des Tubus 1, ein zentrales Lumen 4 und zwei in der Wand des Tubus 1 vorgesehene Wandkanäle 5, die zum Aufblasen oder Entlüften der Manschette dienen. Alternativ dazu kann einer der Wandkanäle 5 auch zum Zuführen oder Absaugen von Flüssigkeit bzw. Sekret dienen, wenn er aus dem Tubus 1 in die Luftröhre mündet.

In den Figuren ist die Manschette im befüllten Zustand dargestellt. In diesem Zustand liegt die dünne Wand der Manschette an der Luftröhre des Patienten an. Dabei kommt es zu Ausbildung mehrerer Längsfalten 3. Die Figuren 1 bis 3 zeigen den Erfindungsgegenstand jedoch nur sehr schematisch und auch die Bildung der Falten 3, die in allen drei Figuren erkennbar sind, ist hier lediglich schematisch wiedergegeben. Die Falten können einen beliebigen Verlauf haben wobei sie sich nicht notwendigerweise über die gesamte Länge der Manschette erstrecken.

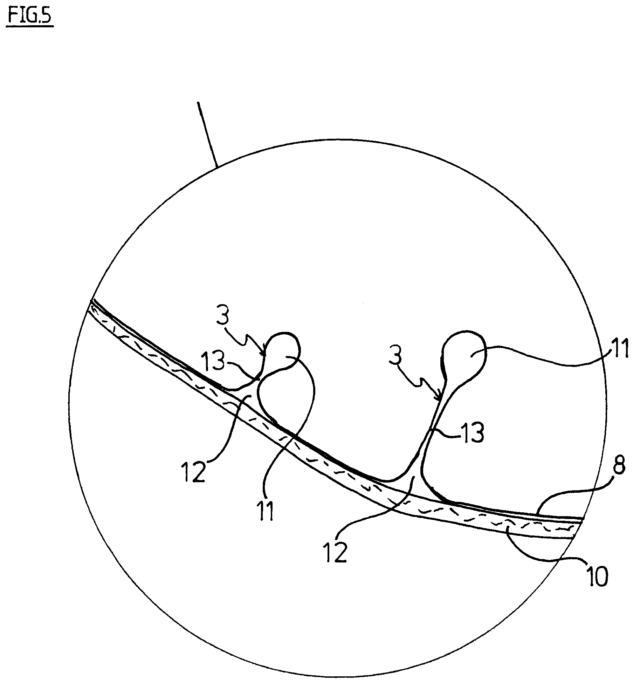

In Figur 5 ist zur besseren Unterscheidung die Folie, aus welcher die Manschette 2 besteht, mit 8 bezeichnet. In Figur 5 ist in starker Vergrößerung und wiederum nur schematisch dargestellt, wie sich die Folie 8 der Manschette 2 an die Wand 10 einer Trachea anlegt.

Während sich in dem faltenfreien Bereich die Folie 2 glatt und dicht an die Wand 10 der Trachea anlegt, ergeben sich im Bereich einer unvermeidbaren Falte 3 vor allen Dingen zwei kritische Durchgänge, die mit 11 und 12 bezeichnet sind. 11 bezeichnet dabei eine Faltenöse, die sich am inneren Ende bzw. Grund einer Falte 3 bildet, wenn das Folienmaterial in einer Schlaufe zurückgeführt wird, so daß sich im Abstand von dem Grund der Falte die Außenwände der Folie aneinander anlegen, wie dies in dem Bereich 13 angedeutet wird, jedoch dort, wo die Folie 2 am Grund der Falte eine 180°-Schleife bildet, die in der Folie wirkenden elastischen Rückstellkräfte verhindern, daß sich ein scharfer Knick ausbildet, so daß die hier nur schematisch dargestellte Faltenöse 11 gebildet wird. Hier nimmt die Folie 8 aufgrund der in ihr wirkenden elastischen Rückstellkräfte nur einen begrenzten Biegeradius an, der bei den meisten bevorzugten Folienmaterialien, wie z. B. Polyurethan, beim Sechs- bis Fünfzehnfachen der Wandstärke des Folienmaterials liegt, jedenfalls bei den hier konkret wirkenden Kräften. Eine ähnlich problematische Zone ist jedoch auch der Zwickelbereich 12, der entsteht, wo die Folie 8 zur Bildung der Falte die Wand der Trachea verläßt und auf der anderen Seite der Falte sich wieder an die Trachea anschmiegt. Dabei ist zu berücksichtigen, daß auf der Innenseite der Manschette lediglich ein geringer Überdruck von etwa 20 - 30 Millibar aufgebracht wird, der so ausgewählt wird, daß sich die Folie 8 zwar abgedichtet an die Wand 10 der Trachea anlegt, die Durchblutung in der Trachealwand 10 jedoch nicht beeinträchtigt werden soll. Demzufolge wirken auch auf die Wand der Manschette im Bereich der Falten entsprechend geringe Kräfte ein, die nicht dazu ausreichen, die elastischen Rückstellkräfte zu überwinden, so daß sich im Ergebnis in etwa die dargestellten geometrischen Verhältnisse ergeben, auch wenn die hier gewählte Darstellung nicht unbedingt maßstabsgetreu ist.

In der dargestellten Ausführungsform ist die Oberfläche der Manschette mit einer fluorhaltigen Verbindung, C₂F₆, beschichtet. Dabei bildet sich eine dünne, polymere Kohlenwasserstoffschicht (CₓF_{y}H_{z})ₙ aus, die an die Manschettenoberfläche kovalent gebunden ist. Diese Beschichtung ist hydrophob, so daß keine Flüssigkeiten oder mit Flüssigkeiten benetzte Feststoffe in und durch die gebildeten Falten eindringen können. Aufgrund der hohen Oberflächenspannung vieler Flüssigkeiten wird nämlich das Eindringen dieser Flüssigkeiten in enge Falten deutlich erschwert. Auf diese Weise ist die Dichtwirkung der Manschette erheblich verbessert und eine Aspiration kann wirksam verhindert werden, da weder Flüssigkeiten noch Feststoffe in die unteren Atemwege gelangen können. Die Beschichtung mit C₂F₆ erfolgt mit Hilfe eines Plasmabeschichtungsverfahrens.

Wie in der Schnittansicht aus Figur 2 dargestellt, ist der Tubus 1 ebenfalls mit einer die Oberflächeneigenschaften verändernden Oberflächenschicht 7 versehen. Diese Beschichtung 7 besteht aus einem hydrophoben und schmutzabweisenden Polymer.

Die in der dargestellten Ausführungsform gezeigte Beschichtung 7 des Tubus 1 ist ein Polysilan. Es wurde bei der Extrusion des Tubus-Körpers dem Kunststoffmaterial zugesetzt und hat sich aufgrund seiner gegenüber dem Tubusmaterial geringeren Oberflächenspannung an der äußeren Oberfläche des Tubus 1 angereichert. Dort bildet es auf dem Innen- und dem Außenlumen eine dünne Oberflächenschicht.

Die in den Figuren 2 und 3 gezeigten Oberflächenschichten 6, 7 sind, um überhaupt eine Darstellung zu ermöglichen, in ihrer Dicke nicht maßstäblich dargestellt. Während die hydrophobe, schmutzabweisende Beschichtung 7 des Tubus 1 eine Dicke von etwa 3 µm aufweist, ist die Beschichtung 6 der Manschette 2 vorzugsweise eine atomare Monolage und nach Möglichkeit nur wenige nm, und insbesondere weniger als 100 nm dick.

In Figur 4 ist schematisch ein Schnitt durch die Wand einer Folie 8 wiedergegeben, wobei das eigentliche Folienmaterial hier als Schicht 20 dargestellt ist, die z. B. eine Dicke zwischen 15 und 30 µm haben kann, wobei auf der Außenseite der Folie, die in Figur 4 unten dargestellt ist, zunächst eine wasserundurchlässige Schicht 14 und zusätzlich eine hydrophobe Schicht 15 aufgebracht sind.

Die Dicke der Schichten 14 und 15 ist tatsächlich im Verhältnis zur Dicke des eigentlichen Folien- bzw. Trägermaterials 20 noch wesentlich geringer als hier dargestellt.

Optional oder als Alternative zu der unter der hydrophoben Schicht 15 aufgebrachten, wasserundurchlässigen Schicht 14, kann diese wasserundurchlässige Schicht 14 auch auf der Innenseite des Folienmaterials 20 aufgebracht sein.

Mit der vorliegenden Erfindung gelingt es trotz eines Durchmessers der Faltenösen 11 im Bereich von etwa 150 - 300 Mikrometer und trotz entsprechender Abmessungen in dem Zwickelbereich 12 einen Sekretdurchfluß zu verhindern aufgrund einer hydrophoben Beschichtung 15, die zusätzlich zu einer wasserundurchlässigen Beschichtung 14 auf der Folie 2 aufgebracht ist.

Grundsätzlich steht dem allerdings nicht entgegen, daß Folienmaterialien und Wandstärken verwendet werden, die noch kleinere Faltenösen 11 oder Zwickelbereiche 12 bilden, um den Durchtritt von Sekret dadurch noch besser zu verhindern.

Die wasserundurchlässige Beschichtung 14 sorgt dabei gleichzeitig dafür, daß auch keinerlei Wasser oder Sekretflüssigkeit durch die Wand der Folie 2 hindurchtritt.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

### Bezugszeichenliste

- 1: Endotracheal- oder Tracheotomietubus
- 2: Manschette
- 3: Falten, Längskanäle
- 4: zentrales Lumen
- 5: Wandkanäle
- 6,7: Oberflächenschichten
- 8: Folie
- 10: Wandeiner Trachea
- 11: Faltenöse
- 12: Zwickelbereich
- 13: Bereich anliegender Außenwände

## Patentansprüche

1. Vorrichtung zur Atemluftzuführung, insbesondere Endotrachealtubus oder Tracheostomiekanüle, mit mindestens einem Tubus und mindestens einer mit einem Fluid befüllbaren und an der Außenseite des Tubus vorgesehenen Manschette zur Abdichtung des Tubus gegenüber der Wand der Luftröhre, **dadurch gekennzeichnet, daß** mindestens die Manschette abschnittsweise eine die Oberfläche des Materials der Vorrichtung verändernde, abriebfeste Oberflächenschicht (6, 7) mit einer Schichtdicke von weniger als 10 µm aufweist, wobei die Manschette eine Kombination aus einer wasserundurchlässigen und hydrophoben Beschichtung aufweist.

2. Vorrichtung nach Anspruch 1, **dadurch gekennzeichnet, daß** die Manschette aus einer flexiblen, dünnwandigen Kunststoffolie besteht.

3. Vorrichtung nach Anspruch 1 oder 2, **dadurch gekennzeichnet, daß** die Kunststoffolie den Tubus in Form eines aufgeweiteten Schlauchabschnittes umgreift und an diesem befestigt ist.

4. Vorrichtung nach Anspruch einem der Ansprüche 1 bis 3, **dadurch gekennzeichnet, daß** die wasserundurchlässige Beschichtung aus einem Material besteht, welches aus der Gruppe der folgenden Substanzen oder Kombinationen hieraus ausgewählt ist: Siliciumdioxid, Metalle, Metalloxide oder andere anorganische Beschichtungen, Silane, Siloxane sowie fluorierte Kohlenwasserstoffe, wie Teflon.

5. Vorrichtung nach einem der Ansprüche 1 bis 4, **dadurch gekennzeichnet, daß** die Manschette auf ihrer dem Tubus abgewandten Außenseite mit dem wasserundurchlässigen Material beschichtet ist.

6. Vorrichtung nach Anspruch 2 oder einem der Ansprüche 3 bis 5 wenn abhängig von Anspruch 2, **dadurch gekennzeichnet, daß** auf der Außenseite der Manschette eine zusätzliche hydrophobisierende Schicht aufgebracht ist.

7. Vorrichtung nach Anspruch 6, **dadurch gekennzeichnet, daß** die hydrophobisierende Schicht aus einem Material besteht, welches ausgewählt ist aus der Gruppe, die besteht aus fluorierten Kohlenwasserstoffen, Silanen oder Siloxanen.

8. Vorrichtung nach Anspruch 2 oder einem der Ansprüche 3 bis 7 wenn abhängig von Anspruch 2, **dadurch gekennzeichnet, daß** das Material der Folie, aus welchem die Manschette besteht, ausgewählt ist aus der Gruppe, die besteht aus Polyurethan, PVC, SEBS und Silikon.

9. Vorrichtung nach Anspruch 2 oder einem der Ansprüche 3 bis 8 wenn abhängig von Anspruch 2, **dadurch gekennzeichnet, daß** die Folie eine Wandstärke von weniger als 100 µm (0,1 mm) und vorzugsweise mehr als 5 µm hat.

10. Vorrichtung nach einem der Ansprüche 1 bis 9, **dadurch gekennzeichnet, daß** die Oberflächenschicht sowohl wasserundurchlässig als auch hydrophob ist.

11. Vorrichtung nach einem der Ansprüche 1 bis 10, **dadurch gekennzeichnet, daß** die Manschettenbeschichtung eine Plasmabeschichtung ist.

12. Vorrichtung nach einem der Ansprüche 1 bis 11, **dadurch gekennzeichnet, daß** die Oberflächenschicht durch Oberflächensegregation eines im Manschettenmaterial enthaltenen Additivs, insbesondere Polymers, vorzugsweise fluorhaltige Polymere, Silane oder Siloxane bzw. deren Copolymeren mit dem Manschettenmaterial erzeugt wird.

13. Vorrichtung nach einem der vorstehenden Ansprüche, **dadurch gekennzeichnet, daß** die Oberflächenschicht einen im Vergleich zu dem Material der Vorrichtung verringerten Reibungskoeffizienten aufweist und/oder schmutzabweisend und/oder selbstreinigend ist.

14. Vorrichtung nach einem der vorstehenden Ansprüche, **dadurch gekennzeichnet, daß** die Dicke der Oberflächenschicht weniger als 5 µm, vorzugsweise weniger als 3 µm und besonders bevorzugt weniger als 1 µm und zum Beispiel zwischen 0,05 µm und 0,5 µm beträgt.

15. Vorrichtung nach einem der vorstehenden Ansprüche, **dadurch gekennzeichnet, daß** die Oberflächenschicht fluorhaltig und/oder silikon-, silan- oder siloxanhaltig ist und/oder die Oberflächenschicht CF₄, C₂F₆, ein Silan, vorzugsweise Tetramethylsilan, oder ein Siloxan ist.

## Claims

1. Device for tracheal respiration, in particular endotracheal tube or tracheostomy cannula, comprising at least one tube and at least one cuff that can be filled with a fluid and that is provided on the outside of the tube to seal off the tube from the trachea wall, **characterized in that** at least the cuff has in sections an abrasion-resistant surface layer (6, 7) with a layer thickness of less than 10 µm which modifies the surface of the material of the device, wherein the cuff comprises a combination of a water-impermeable and hydrophobic coating.

2. Device according to claim 1, **characterized in that** the cuff is composed of a flexible, thin-walled plastic film.

3. Device according to claim 1 or 2, **characterized in that** the plastic film surrounds the tube in the shape of a widened section of hose and is secured to same.

4. Device according to one of claims 1 to 3, **characterized in that** the water-impermeable coating is composed of a material which is selected from the group of the following substances or combinations thereof: silicon dioxide, metals, metal oxides or other inorganic coatings, silanes, siloxanes as well as fluorinated hydrocarbons, such as Teflon.

5. Device according to one of claims 1 to 4, **characterized in that** the cuff is coated with the water-impermeable material on its outside facing away from the tube.

6. Device according to claim 2 or one of claims 3 to 5 if dependent on claim 2, **characterized in that** an additional hydrophobing layer is applied to the outside of the cuff.

7. Device according to claim 6, **characterized in that** the hydrophobing layer is composed of a material which is selected from the group comprising fluorinated hydrocarbons, silanes or siloxanes.

8. Device according to claim 2 or one of claims 3 to 7 if dependent on claim 2, **characterized in that** the material of the film of which the cuff is composed is selected from the group comprising polyurethane, PVC, SEBS and silicone.

9. Device according to claim 2 or one of claims 3 to 8 if dependent on claim 2, **characterized in that** the film has a wall thickness of less than 100 µm (0.1 mm) and preferably more than 5 µm.

10. Device according to one of claims 1 to 9, **characterized in that** the surface layer is both water-impermeable and hydrophobic.

11. Device according to one of claims 1 to 10, **characterized in that** the cuff coating is a plasma coating.

12. Device according to one of claims 1 to 11, **characterized in that** the surface layer is produced by surface segregation of an additive contained in the cuff material, in particular polymer, preferably fluorine-containing polymers, silanes or siloxanes or their copolymers with the cuff material.

13. Device according to one of the preceding claims, **characterized in that** the surface layer has a reduced coefficient of friction compared with the material of the device and/or is dirt-repellent and/or self-cleaning.

14. Device according to one of the preceding claims, **characterized in that** the thickness of the surface layer is less than 5 µm, preferably less than 3 µm and particularly preferably less than 1 µm and for example between 0.05 µm and 0.5 µm.

15. Device according to one of the preceding claims, **characterized in that** the surface layer contains fluorine and/or silicone, silane or siloxane and/or the surface layer is CF₄, C₂F₆, a silane, preferably tetramethylsilane, or a siloxane.

## Revendications

1. Dispositif d'amenée d'air respiratoire, notamment tube endotrachéal ou canule de trachéotomie, avec au moins un tube et au moins une manchette adaptée pour pouvoir être remplie d'un fluide et prévue sur la face extérieure du tube pour rendre le tube étanche par rapport à la paroi de la trachée, **caractérisé en ce qu'**au moins la manchette comprend, par zones, une couche de surface (6, 7) résistante à l'abrasion avec une épaisseur de couche inférieure à 10 µm et modifiant la surface du matériau du dispositif, la manchette comprenant une combinaison d'un revêtement imperméable et hydrophobe.

2. Dispositif selon la revendication 1, **caractérisé en ce que** la manchette consiste en un film de matière synthétique flexible à paroi mince.

3. Dispositif selon la revendication 1 ou 2, **caractérisé en ce que** le film de matière synthétique entoure le tube sous la forme d'un tronçon de tuyau élargi et est fixé sur celui-ci.

4. Dispositif selon l'une des revendications 1 à 3, **caractérisé en ce que** le revêtement imperméable consiste en un matériau qui est choisi dans le groupe des substances ou combinaisons suivantes : dioxyde de silicium, métaux, oxydes de métal ou autres revêtements inorganiques, silanes, siloxanes et hydrocarbures fluorés tels que du Téflon.

5. Dispositif selon l'une des revendications 1 à 4, **caractérisé en ce que** la manchette est revêtue, sur sa face extérieure opposée au tube, du matériau imperméable.

6. Dispositif selon la revendication 2 ou une des revendications 3 à 5 si rattachées à la revendication 2, **caractérisé en ce que**, sur la face extérieure de la manchette, une couche supplémentaire rendant hydrophobe est appliquée.

7. Dispositif selon la revendication 6, **caractérisé en ce que** la couche rendant hydrophobe consiste en un matériau choisi dans le groupe formé par des hydrocarbures fluorés, des silanes ou siloxanes.

8. Dispositif selon la revendication 2 ou une des revendications 3 à 7 si rattachées à la revendication 2, **caractérisé en ce que** le matériau du film en lequel la manchette consiste, est choisi dans le groupe qui est formé par polyuréthane, PVC, SEBS et silicone.

9. Dispositif selon la revendication 2 ou une des revendications 3 à 8 si rattachées à la revendication 2, **caractérisé en ce que** le film présente une épaisseur de paroi de moins de 100 µm (0,1 mm) et de préférence plus que 5 µm.

10. Dispositif selon l'une des revendications 1 à 9, **caractérisé en ce que** la couche de surface est aussi bien imperméable qu'hydrophobe.

11. Dispositif selon l'une des revendications 1 à 10, **caractérisé en ce que** le revêtement de manchette est un revêtement par plasma.

12. Dispositif selon l'une des revendications 1 à 11, **caractérisé en ce que** la couche de surface est réalisée par ségrégation de surface d'un additif contenu dans le matériau de la manchette, notamment d'un polymère, de préférence des polymères fluorés, des silanes ou siloxanes ou des copolymères de ceux-ci, avec le matériau de la manchette.

13. Dispositif selon l'une des revendications précédentes, **caractérisé en ce que** la couche de surface présente un coefficient de friction réduit comparé au matériau du dispositif et/ou repousse la saleté et/ou est auto-nettoyant.

14. Dispositif selon l'une des revendications précédentes, **caractérisé en ce que** l'épaisseur de la couche de surface est de moins de 5 µm, de préférence de moins de 3 µm et de manière particulièrement préférée de moins de 1 µm et par exemple entre 0,05 µm et 0,5 µm.

15. Dispositif selon l'une des revendications précédentes, **caractérisé en ce que** la couche de surface contient du fluor et/ou du silicone, du silane ou du siloxane et/ou que la couche de surface est du CF₄, du C₂F₆, un silane, de préférence du tétraméthylsilane, ou un siloxane.
